# EUROPÄISCHE PATENTSCHRIFT

(11) **EP 1 919 405 B1**
(45) Veröffentlichungstag und Bekanntmachung des Hinweises auf die Patenterteilung: **05.10.2011**
(21) Anmeldenummer: 06776680.8
(22) Anmeldetag: 08.08.2006
(51) Int. Cl.: A61F 2/46

(54) **INSTRUMENT ZUR HANDHABUNG EINER GELENKKOMPONENTE EINER GELENKPROTHESE**
INSTRUMENT FOR HANDLING A JOINT COMPONENT OF A JOINT PROSTHESIS
INSTRUMENT POUR MANIPULER UNE COMPOSANTE ARTICULAIRE D'UNE PROTHESE ARTICULAIRE

(30) Priorität: 30.08.2005 DE 102005041062
(43) Veröffentlichungstag der Anmeldung: 14.05.2008
(73) Patentinhaber: Smith & Nephew Orthopaedics AG, 6343 Rotkreuz (CH)
(72) Erfinder: IMHOF, Martin, 6045 Meggen (CH); BÜTLER, Beat, 6312 Steinhausen (CH); BRACK, René, 6330 Cham (CH)
(74) Vertreter: Popp, Eugen
(86) Internationale Anmeldenummer: PCT/EP2006/007841
(87) Internationale Veröffentlichungsnummer: WO 2007/025639

(56) Entgegenhaltungen:
- DE-A1- 10 128 234
- DE-A1- 19 704 577
- DE-A1- 19 722 923
- DE-U1- 29 823 658

## Beschreibung

Die Erfindung betrifft ein Instrument zur Handhabung einer eine Gelenkfläche umfassenden Gelenkkomponente einer Gelenkprothese, mit einem Griffteil, einem an die Gelenkfläche abdichtend anlegbaren Saugelement in Form eines Saugnapfes mit Entlüftungsöffnung, und einem der Entlüftungsöffnung des Saugnapfes zugeordneten Druckentlastungsventil. Ein derartiges Instrument ist zum Beispiel aus der DE 101 28 234 A1 bekannt. Diese bekannte Konstruktion zeichnet sich durch eine relativ unhandliche Mechanik für die Aufhebung eines zum Setzen oder Entfernen der Gelenkkomponente erforderlichen Unterdrucks in dem zwischen Gelenkfläche und Saugnapf begrenzten Saugraum aus. Vor allem besteht bei der Handhabung des bekannten Instruments die Gefahr, daß ungewollt der Unterdruck im erwähnten Saugraum aufgehoben wird mit der Folge, daß die Gelenkkomponente in einem unerwünschten Moment vom Instrument fällt. Der Grund dafür liegt vornehmlich darin, daß das Betätigungselement für das Druckentlastungsventil axial über die Grifffläche nach hinten vorsteht, das heißt in Richtung zum Benutzer hin. Somit kann nicht ausgeschlossen werden, daß beim Greifen des Instruments versehentlich das Betätigungselement für das Druckentlastungsventil berührt und unerwünscht betätigt wird.

Außerdem ist eine sichere Betätigung nur unter Verwendung von zwei Händen möglich.

Ähnlich verhält es sich bei dem Setzgerät gemäß der DE 197 22 923 A1. Auch dort ist eine Zweihand-Bedienung zwingend.

Der vorliegenden Erfindung liegt die Aufgabe zugrunde, ein Instrument der eingangs genannten Art derart weiterzubilden, daß eine einfache und funktionssichere EinhandBedienung möglich ist. Das erfindungsgemäße Instrument soll sich insbesondere durch eine Ergonomie auszeichnen.

Diese Aufgabe wird erfindungsgemäß durch die kennzeichnenden Merkmale des Anspruches 1 gelöst, wobei vorteilhafte Weiterbildungen und konstruktive Details sowie Alternativen in den Unteransprüchen beschrieben sind.

Ein wesentlicher Punkt der Erfindung liegt unter anderem darin, daß das Druckentlastungsventil mit einem im Bereich der Grifffläche des Griffteils beweglich angeordneten und beim Greifen mit einer Hand durch einen Finger oder Daumen derselben betätigbaren Betätigungselement gekoppelt ist. Natürlich liegt dieses Betätigungselement auf Höhe einer Finger-, insbesondere Zeigefinger- oder Daumenkuppe, das heißt vom Benutzer aus betrachtet distalen Bereich der Grifffläche. Ist das Griffteil als Rundgriff ausgebildet, wird es mit einer Hand schlicht umgriffen, wobei sich das Betätigungselement dann in vorbestimmtem Abstand von Daumen und Zeigefinger befindet, vorzugsweise etwa der halben Länge von Zeigefinger oder Daumen. Damit ist das Betätigungselement ohne jegliche Verrenkungen betätigbar. Durch Betätigung des Betätigungselements soll das Ventil von der Entlüftungsöffnung abhebbar sein, so daß Umgebungsluft in den durch Saugnapf einerseits und Gelenkfläche andererseits begrenzten Saugraum eindringen kann mit der Folge, daß dann das Instrument von der Gelenkfläche freikommt.

Das Betätigungselement kann als Schieber oder Kipphebel ausgebildet sein. Des weiteren ist das Betätigungselement vorzugsweise gegen die Wirkung einer elastischen Vorspannung auf das Druckentlastungsventil und/oder das Betätigungselement selbst betätigbar. Dies bedeutet, daß das Druckentlastungsventil in Schließstellung elastisch vorgespannt ist. Diese elastische Vorspannung soll durch das Betätigungselement aufgehoben werden können.

Weitere konstruktive Einzelheiten sind in den Ansprüchen 4 ff beschrieben, wobei Anspruch 11 eine Alternative betrifft dergestalt, daß das Betätigungselement als Schieber ausgebildet ist, der sich im Bereich der Grifffläche befindet und sich parallel dazu verschieben läßt.

Nachstehend wird eine bevorzugte Ausführungsform eines erfindungsgemäß ausgebildeten Instruments anhand der beigefügten Zeichnung näher erläutert. Diese zeigt in:
- Fig. 1: ein stabförmiges Instrument gemäß vorliegender Erfindung in Seiten- ansicht; und
- Fig. 2: das Instrument gemäß Fig. 1 im Längsschnitt.

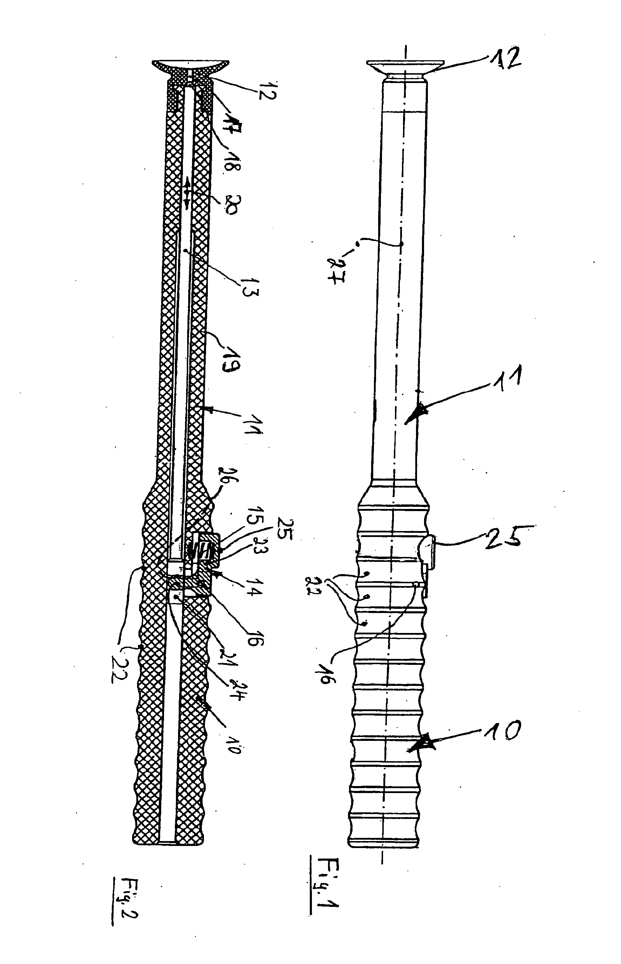

In den Figuren 1 und 2 ist ein stabartiges Instrument zur Handhabung einer eine Gelenkfläche umfassenden Gelenkkomponente einer Gelenkprothese, zum Beispiel Hüftgelenkprothese, in Seitenansicht und im Längsschnitt dargestellt. Das Instrument ist in den Figuren 1 und 2 mit der Bezugsziffer 11 gekennzeichnet. Es umfaßt ein Griffteil 10, ein an die hier nicht dargestellte Gelenkfläche abdichtend anlegbares Saugelement in Form eines Saugnapfes 12 mit Entlüftungsöffnung 17, und ein dieser Entlüftungsöffnung 17 zugeordnetes Druckentlastungsventil 18. Das Druckentlastungsventil 18 ist Teil eines Ventilstabes 13. Dieser Ventilstab 13 ist innerhalb eines zwischen Saugnapf 12 und Griffteil angeordneten Hohlstabes 19 axial verschieblich gelagert (Doppelpfeil 20 in Fig. 2).

Das dem Saugnapf 12 abgewandte Ende 21 des Ventilstabes 13 ist mit einem im Bereich der Grifffläche 22 des Griffteils 10 beweglich angeordneten und beim Greifen mit einer Hand durch einen Finger oder Daumen derselben betätigbaren Betätigungselement 14 gekoppelt derart, daß bei Betätigung des Betätigungselements 14 der Ventilstab 13 und damit das Ventil 18 von der Entlüftungsöffnung 17 des Saugnapfes 12 abhebbar ist. In diesem Fall wird der Ventilstab 13 in Fig. 2 nach rechts bewegt. Bei der dargestellten Ausführungsform ist das Betätigungselement 14 als Kipphebel ausgebildet, und zwar konkret als doppelarmiger Kipphebel. Dieser ist um eine sich quer zur Längsrichtung des Instruments 11 erstreckende Achse 16 verschwenkbar gelagert. Des weiteren ist dieser Kipphebel 14 gegen die Wirkung einer elastischen Vorspannung, hier einer Schraubendruckfeder 15, betätigbar. Wie bereits erwähnt, ist der im Bereich des Griffteils bzw. der Grifffläche 22 desselben verschwenkbar gelagerte Kipphebel 14 doppelarmig ausgebildet, wobei der eine Arm 24 mit dem Ventilstab 13 in Wirkverbindung steht und der andere Arm 23 eine druckknopfartige Betätigungsfläche 25 aufweist, die in Richtung quer zur Grifffläche 22 bewegbar ist. Die erwähnte elastische Vorspannung des Betätigungselements 14 ist durch die zwischen druckknopfartiger Betätigungsfläche 25 und Griffteil 10 angeordnete Druckfeder 15 erhalten. Die beiden Arme 23, 24 des doppelarmigen Kipphebels 14 stehen senkrecht zueinander, wobei die Lagerung des Kipphebels um die Achse 16 derart ist, daß der eine die druckknopfartige Betätigungsfläche 25 umfassende Arm 23 sich etwa parallel zur Grifffläche 22 bzw. zur Längsrichtung des Instruments 11, und der andere, mit dem Druckentlastungsventil 18 bzw. Ventilstab 13 in Wirkverbindung stehende Arm 24 sich etwa senkrecht zur Grifffläche 22 in diese hineinerstreckt. Der mit dem Ventilstab 13 in Wirkverbindung stehende Arm 24 des Kipphebels 14 greift in eine korrespondierende Öffnung, nämlich Querbohrung 26 des Ventilstabes 13 ein. Dementsprechend wird durch Ausübung von Druck auf die Betätigungsfläche 25 des Kipphebels 14 diese in die Grifffläche 22 hineinbewegt. Gleichzeitig wird dadurch der sich senkrecht zur Grifffläche 22 erstreckende Arm 24 des Kipphebels in Fig. 2 nach rechts bewegt unter entsprechender Mitnahme des Ventilstabes 13 sowie des am distalen Ende desselben angeordneten Ventils 18, wodurch die Entlüftungsöffnung 17 freigegeben wird. Es findet dann ein Druckausgleich zwischen dem zwischen Gelenkfläche und Saugnapf definierten Raum einerseits und der äußeren Umgebung andererseits statt, und zwar durch den Hohlstab 19 hindurch. Zu diesem Zweck ist der Ventilstab vorzugsweise innerhalb des Instruments bzw. des Hohlstabes 19 mit radialem Spiel gelagert. Es können jedoch auch sich axial erstreckende Luftkanäle zwischen Ventilstab 13 und Hohlstab 19 ausgebildet sein, die bei Öffnung des Ventils 18 einen Druckausgleich zwischen dem zwischen Gelenkfläche und Saugnapf definierten Saugraum einerseits und Umgebung andererseits ermöglichen. Die Öffnung zur äußeren Umgebung erfolgt dabei entweder im Bereich der Lagerung des Kipphebels 14 oder axial durch den Griffteil 10 hindurch.

Wie bereits eingangs erwähnt, kann alternativ der Ventilstab 13 im Bereich der Grifffläche 22 mit einem mit dieser bündigen oder diese geringfügig überragenden Schieber verbunden sein, der in Richtung der axialen Erstreckung des Ventilstabes 13 und etwa parallel zur Grifffläche 22 gegen die Wirkung eines elastischen Elements verschiebbar ist derart, daß dadurch das Druckentlastungsventil 18 die Entlüftungsöffnung 17 freigibt.

Der zum Setzen oder Entfernen einer Gelenkkomponente bzw. eines sogenannten Inlays erforderliche Unterdruck zwischen Inlay und Instrument wird dadurch erreicht, daß das Instrument mit dem Saugnapf 12 gegen die Gelenkfläche gedrückt wird. Dadurch wird ein Teil der sich im Raum zwischen Gelenkfläche und Saugnapf 12 befindlichen Luft aus diesem herausgepreßt, und zwar am äußeren Umfang des Saugnapfes 12 vorbei. Bedingt durch die Eigenelastizität des Saugnapfes 12 versucht dieser das Instrument 11 von der Gelenkfläche wieder wegzudrücken. Dadurch entsteht in dem durch die Gelenkfläche einerseits und den Saugnapf 12 begrenzten Raum ein Unterdruck, durch den das Instrument an der Gelenkfläche und damit am Inlay gehalten wird. Das Inlay läßt sich durch das Instrument 11 bequem manipulieren, das heißt setzen, verdrehen oder auch wieder entfernen. Um den erwähnten Unterdruck aufzuheben, muß der erwähnte Saugraum zur Umgebung hin geöffnet werden. Dies erfolgt in der beschriebenen Weise durch die Entlüftungsöffnung 17 hindurch.

Zur Gelenkachse 16 des Kipphebels 14 sei noch erwähnt, daß diese sich in radialem Abstand von der Instrumenten-Längsmittenachse 27 erstreckt, und zwar näher zur Grifffläche 22 des Griffteils 10 als zur Längsmittenachse 27. Die Betätigungsfläche 25 des Kipphebels 14 ist darüber hinaus im distalen Bereich des Griffteils 10 zugänglich, so daß die Betätigung beim Umgreifen des Griffteils 10 mit einer Hand problemlos, das heißt ohne Verrenkungen entweder mit dem Daumen oder dem Zeigefinger erfolgen kann.

Griffteil 10 und Hohlstab bzw. Hohlschaft 19 sind bei der dargestellten Ausführungsform einteilig, zum Beispiel aus Aluminium hergestellt. Am distalen Ende des Hohlzylinders bzw. -schaftes 19 ist der Saugnapf 12 angebracht, und zwar in herkömmlicher Weise übergestülpt (siehe dazu DE 101 28 234 A1 oder auch DE 197 08 604 C1). Der Kipphebel 14 kann aus demselben Material wie Griffteil 10 und Hohlzylinder bzw. -schaft 19 bestehen, das heißt vorzugsweise ebenfalls aus Aluminium. Der Ventilstab 13 samt Ventil 18 besteht ebenso wie die Schwenkachse 16 vorzugsweise aus einem härteren Material, zum Beispiel Edelstahl.

Das distale Ende des Ventilstabes 13, der das Druckentlastungsventil 18 definiert, ist etwa semi-sphärisch, und damit selbstzentrierend relativ zur zugeordneten Entlüftungsöffnung 17 ausgebildet. Der Saugnapf 12 besteht aus einem hochelastischen Material, zum Beispiel Silikongummi oder dgl. Auch der dem Druckentlastungsventil 18 zugeordnete Ventilsitz an dem dem Druckentlastungsventil 18 zugeordneten Ende der Entlüftungsöffnung 17 ist somit aus diesem weichelastischen Material hergestellt, wodurch eine hohe Dichtigkeit in Schließstellung des Druckentlastungsventils 18 bzw. des zugeordneten Ventilstabes 13 erreicht wird.

### Bezugszeichen

- 10: Griffteil
- 11: Instrument
- 12: Saugnapf
- 13: Ventilstab
- 14: Betätigungselement (Kipphebel)
- 15: Druckfeder
- 16: Schwenkachse
- 17: Entlüftungsöffnung (Entlüftungsbohrung)
- 18: Druckentlastungsventil
- 19: Hohlstab bzw. -schaft
- 20: Doppelpfeil
- 21: proximales Ende des Ventilstabes
- 22: Grifffläche
- 23: Kipphebelarm
- 24: Kipphebelarm
- 25: druckknopfartige Betägigungsfläche
- 26: Querbohrung
- 27: Längsmittenachse

## Patentansprüche

1. Instrument (11) zur Handhabung einer eine Gelenkfläche umfassenden Gelenkkomponente einer Gelenkprothese, mit einem Griffteil (10), einem an die Gelenkfläche abdichtend anlegbaren Saugelement in Form eines Saugnapfes (12) mit Entlüftungsöffnung (17), und einem der Entlüftungsöffnung (17) des Saugnapfes (12) zugeordneten Druckentlastungsventil (18),
**dadurch gekennzeichnet, daß**
das Druckentlastungsventil (18) mit einem im Bereich der Grifffläche (22) des Griffteils (10) beweglich angeordneten und beim Greifen mit einer Hand durch einen Finger oder Daumen derselben betätigbaren Betätigungselement (14) gekoppelt ist derart, daß bei Betätigung des Betätigungselements (14) das Ventil (18) von der Entlüftungsöffnung (17) abhebbar ist.

2. Instrument nach Anspruch 1,
**dadurch gekennzeichnet, daß**
das Betätigungselement (14) als Schieber oder Kipphebel ausgebildet ist.

3. Instrument nach Anspruch 1 oder 2,
**dadurch gekennzeichnet, daß**
das Betätigungselement (14) gegen die Wirkung einer elastischen Vorspannung auf das Druckendastungsventil (18) und/oder das Betätigungselement (14) selbst betätigbar ist.

4. Instrument nach einem der Ansprüche 1 bis 3,
**dadurch gekennzeichnet, daß**
das Druckentlastungsventil (18) Teil eines mit dem Betätigungselement (14) gekoppelten Ventilstabes (13) ist, der zwischen Betätigungselement (14) und Entlüftungsöffnung (17) axial verschieblich (Doppelpfeil 20) gelagert ist.

5. Instrument nach Anspruch 4,
**dadurch gekenntzeichnet, daß**
es zwischen Griffteil (10) und Saugnapf (12) einen Hohlstab bzw. -schaft (19) umfaßt, innerhalb dem der Ventilstab (13) axial verschieblich gelagert ist.

6. Instrument nach einem der Ansprüche 2 bis 5,
**dadurch gekennzeichnet, daß**
der im Bereich der Grifffläche (22) verschwenkbar gelagerte Kipphebel (14) doppelarmig ausgebildet ist, wobei der eine Arm (24) mit dem Druckentlastungsventil (18), insbesondere Ventilstab (13) in Wirkverbindung steht, während der andere Arm (23) eine druckknopfartige Betätigungsfläche (25) aufweist, die in Richtung quer zur Grifffläche (22) bewegbar ist.

7. Instrument nach Anspruch 6,
**dadurch gekennzeichnet, daß**
die elastische Vorspannung des Betätigungselements (14) durch eine zwischen druckknopfartiger Betätigungsfläche (25) und Griffteil (10) angeordnete Druckfeder (15) erhalten ist.

8. Instrument nach Anspruch 6 oder 7,
**dadurch gekennzeichnet, daß**
die beiden Arme (23, 24) des doppelarmigen Kipphebels (14) etwa senkrecht zueinander stehen, und daß die Lagerung dieses Kipphebels (14) derart ist, daß der eine die druckknopfartige Betätigungsfläche (25) umfassende Arm (23) sich etwa parallel zur Grifffläche (22) und der andere, mit dem Druckentlastungsventil (18), insbesondere Ventilstab (13) in Wirkverbindung stehende Arm (24) sich etwa senkrecht zur Grifffläche (22) in diese hineinerstreckt.

9. Instrument nach Anspruch 8,
**dadurch gekennzeichnet, daß**
der mit dem Ventilstab (13) in Wirkverbindung stehende Arm (24) des Kipphebels (14) in eine korrespondierende Öffnung, insbesondere Querbohrung (26) des Ventilstabes (13) eingreift.

10. Instrument nach einem der Ansprüche 1 bis 9,
**dadurch gekennzeichnet, daß**
beim Abheben des Druckentlastungsventils (18) eine Fluidverbindung zwischen Entlüftungsöffnung (17) und äußerer Umgebung freigegeben ist.

11. Instrument nach einem der Ansprüche 1 bis 5 und/oder 10,
**dadurch gekenntzeichnet, daß**
der Ventilstab (13) im Bereich der Grifffläche (22) mit einem mit dieser bündigen oder diese geringfügig überragenden Schieber verbunden ist, der in Richtung der axialen Erstreckung des Ventilstabes (13) und etwa parallel zur Grifffläche (22) gegen die Wirkung eines elastischen Elements vorschiebbar ist derart, daß **dadurch** das Druckentlastungsventil (18) die Entlüftungsöffnung (17) freigibt.

## Claims

1. Instrument (11) for handling a joint component of a joint prosthesis, whichjoint component includes a joint surface, the instrument having a grip portion (10), a suction element, in the form of a suction cup (12) with an air-vent opening (17), placeable in a sealing manner against the joint surface, and a pressure-relief valve (18) associated with the air-vent opening (17) of the suction cup (12),
**characterised in that**
the pressure-relief valve (18) is coupled to an actuating element (14) which is arranged so as to be movable in the region of the grip surface (22) of the grip portion (10) and, when gripped with one hand, is actuable with a finger or the thumb of that hand, so that on actuation of the actuating element (14) the valve (18) is liftable away from the air-vent opening (17).

2. Instrument according to claim 1,
**characterised in that**
the actuating element (14) is in the form of a slider or a rocker arm.

3. Instrument according to claim 1 or 2,
**characterised in that**
the actuating element (14) is actuable against the action of a resilient bias on the pressure-relief valve (18) and/or on the actuating element (14) itself.

4. Instrument according to any one of claims 1 to 3,
**characterised in that**
the pressure-relief valve (18) is part of a valve stem (13) which is coupled to the actuating element (14), which valve stem is mounted so as to be axially displaceable (double-headed arrow 20) between the actuating element (14) and the air-vent opening (17).

5. Instrument according to claim 4,
**characterised in that**
it comprises, between the grip portion (10) and the suction cup (12), a hollow rod or shaft (19), inside which the valve stem (13) is mounted so as to be axially displaceable.

6. Instrument according to any one of claims 2 to 5,
**characterised in that**
the rocker arm (14), which is mounted so as to be pivotable in the region of the grip surface (22), is of double-armed construction, one arm (24) being in operative connection with the pressure-relief valve (18), especially the valve stem (13), while the other arm (23) has a push-button-like actuating surface (25) which is movable in a direction transverse to the grip surface (22).

7. Instrument according to claim 6,
**characterised in that**
the resilient bias of the actuating element (14) is produced by a compression spring (15) arranged between the push-button-like actuating surface (25) and the grip portion (10).

8. Instrument according to claim 6 or 7,
**characterised in that**
the two arms (23, 24) of the double-armed rocker arm (14) are approximately perpendicular to one another, and **in that** the rocker arm (14) is mounted so that one arm (23), which includes the push-button-like actuating surface (25), extends approximately parallel to the grip surface (22) and the other arm (24), which is in operative connection with the pressure-relief valve (18), especially the valve stem (13), extends into the grip surface (22) approximately perpendicularly thereto.

9. Instrument according to claim 8,
**characterised in that**
the arm (24) of the rocker arm (14) in operative connection with the valve stem (13) engages in a corresponding opening, especially a transverse bore (26) of the valve stem (13).

10. Instrument according to any one of claims 1 to 9,
**characterised in that**
when the pressure-relief valve (18) is lifted, a fluid connection is unblocked between the air-vent opening (17) and the external environment.

11. Instrument according to any one of claims 1 to 5 and/or 10,
**characterised in that**
the valve stem (13) is joined in the region of the grip surface (22) to a slider which is flush with or projects very slightly above the grip surface, which slider is displaceable in the direction of the axial run of the valve stem (13) and approximately parallel to the grip surface (22) against the action of a resilient element, with the result that the pressure-relief valve (18) unblocks the air-vent opening (17).

## Revendications

1. Instrument (11) pour manipuler une composante d'articulation, comprenant une surface articulée, d'une prothèse articulaire, comportant un manche (10), un élément d'aspiration en forme de ventouse (12), destinée à être appliquée de manière étanche sur la surface articulée et munie d'une ouverture d'échappement d'air (17), et une soupape de détente (18) associée à l'ouverture d'échappement d'air (17) de la ventouse (12), **caractérisé en ce qu'**un élément de manoeuvre (14), disposé mobile dans la zone de la surface de préhension (22) du manche (10) et destiné à être actionné par un doigt ou le pouce d'une main lorsqu'il est saisi par une main, est couplé à la soupape de détente (18), de telle sorte qu'en cas d'actionnement de l'élément de manoeuvre (14), la soupape (18) peut se retirer de l'ouverture d'échappement d'air (17).

2. Instrument selon la revendication 1, **caractérisé en ce que** l'élément de manoeuvre (14) est réalisé sous la forme d'un curseur ou d'un levier basculant.

3. Instrument selon la revendication 1 ou 2, **caractérisé en ce que** l'élément de manoeuvre (14) peut être actionné dans le sens opposé à l'action d'une précontrainte élastique exercée sur la soupape de détente (18) et/ou sur l'élément de manoeuvre (14) lui-même.

4. Instrument selon l'une quelconque des revendications 1 à 3, **caractérisé en ce que** la soupape de détente (18) est une partie d'une tige de soupape (13), qui est couplée à l'élément de manoeuvre (14) et qui est logée entre l'élément de manoeuvre (14) et l'ouverture d'échappement d'air (17) de manière mobile axialement (double flèche 20).

5. Instrument selon la revendication 4, **caractérisé en ce que**, entre le manche (10) et ventouse (12), il comprend une tige creuse (19), à l'intérieur de laquelle la tige de soupape (13) est logée de manière mobile axialement.

6. Instrument selon l'une quelconque des revendications 2 à 5, **caractérisé en ce que** le levier basculant (14), monté pivotant dans la zone de la surface de préhension (22), est un levier à deux bras, dont un bras (24) coopère avec la soupape de détente (18), en particulier la tige de soupape (13), tandis que l'autre bras (23) comporte une surface de manoeuvre (25) en forme de bouton poussoir, qui est mobile dans la direction transversalement à la surface de préhension (22).

7. Instrument selon la revendication 6, **caractérisé en ce que** la précontrainte élastique de l'élément de manoeuvre (14) est obtenue par un ressort de pression (15), disposé entre la surface de manoeuvre (25) en forme de bouton poussoir et le manche (10).

8. Instrument selon la revendication 6 ou 7, **caractérisé en ce que** les deux bras (23, 24) du levier basculant (14) à deux bras sont sensiblement perpendiculaires l'un à l'autre, et **en ce que** ledit levier basculant (14) est monté de telle sorte que le bras (23), muni de la surface de manoeuvre (25) en forme de bouton poussoir, s'étend sensiblement parallèlement à la surface de préhension (22), et l'autre bras (24), qui coopère avec la soupape de détente (18), en particulier la tige de soupape (13), s'étend sensiblement perpendiculairement à la surface de préhension (22) vers l'intérieur de celle-ci.

9. Instrument selon la revendication 8, **caractérisé en ce que** le bras (24) du levier basculant (14), lequel coopère avec la tige de soupape (13), s'engage dans une ouverture correspondante, en particulier une forure transversale (26) de la tige de soupape (13).

10. Instrument selon l'une quelconque des revendications 1 à 9, **caractérisé en ce que**, sous l'effet du retrait de la soupape de détente (18), une liaison fluidique est établie entre l'ouverture d'échappement d'air (17) et l'environnement extérieur.

11. Instrument selon l'une quelconque des revendications 1 à 5 et/ou 10, **caractérisé en ce que** la tige de soupape (13), dans la zone de la surface de préhension (22), est reliée à un curseur, situé à fleur de cette dernière ou légèrement en saillie par rapport à cette dernière, lequel est apte à être déplacé dans la direction de l'extension axiale de la tige de soupape (13) et sensiblement parallèlement à la surface de préhension (22), dans le sens opposé à l'action d'un élément élastique, de telle sorte que, de ce fait, la soupape de détente (18) libère l'ouverture d'échappement d'air (17).
